# EUROPEAN PATENT APPLICATION

(11) **EP 3 199 239 A1**
(43) Date of publication of application: **02.08.2017**
(21) Application number: 16000199.6
(22) Date of filing: 27.01.2016
(51) Int. Cl.: B01L 3/00

(54) **METHOD AND DEVICE FOR SPATIALLY CONTROLLED PARALLEL TRANSFECTION - FLOATING WELLS**

(71) Applicant: Baden-Württemberg Stiftung gGmbH, 70174 Stuttgart (DE)
(72) Inventor: Erfle, Holger, Dr., 69239 Neckarsteinach (DE); Lisauskas, Tautvydas, Dr., 69126 Heidelberg (DE); Starkuviene, Vytaute, Dr., 69239 Neckarsteinach (DE)
(74) Representative: Müller-Boré & Partner Patentanwälte PartG mbB

(57) **Abstract**

The present invention relates to a cell array comprising a solid carrier comprising on a surface a plurality of spots having a spot size X, and cells on the surface of the solid carrier, characterized in that the solid carrier comprises 9 to 400 spots per cm², a manufacturing method of a cell array and the use of the cell array for solid phase transfection.

## Description

The present invention relates to a cell array comprising a solid carrier comprising on a surface a plurality of spots having a spot size X, and cells on the surface of the solid carrier, characterized in that the solid carrier comprises 9 to 400 spots per cm², a manufacturing method of a cell array and the use of the cell array for solid phase transfection.

Cell arrays and specifically high-density cell arrays (HD-CA) represent a technology intended to perform tests (screening) of many different component effects on cells in a single well format. Transfection reagents are distributed on a solid carrier and dried. Adherent cells are plated (seeded) on top of the solid carrier. At sites of spotted transfection reagents, cells are transfected with respective reagents.
Homogeneous cell and transfection material distribution is of crucial importance for the comparative analysis of multiple data points in an unbiased and position-independent way. Generally, however, cells and transfection material are not homogeneously distributed in a well. In particular, at the borders of the well and in the centre of said well the density of the cells and of the transfection material is high and in-between both regions the density of cells and of the transfection material is low. Reasons for this phenomenon may be vibrations, material transport, i.e. cell and transfection material transport, caused by the coffee ring and Marangoni effects and bending of the solid carrier due to the weight of the media on top of said carrier.

Thus, the problem underlying the present invention is to provide a cell array having a homogenous cell and transfection material distribution and a manufacturing method of a cell array, wherein the cells and the transfection material are homogeneously distributed. A further object of the present invention is to provide a use of the cell array for solid phase transfection.

The solution to the above technical problem is achieved by the embodiments characterized in the claims.

In particular, the present invention relates to a cell array comprising a solid carrier comprising on a surface a plurality of spots having a spot size X, and cells on the surface of the solid carrier, characterized in that the solid carrier comprises 9 to 400 spots per cm².

According to a preferred embodiment of the present invention, the cells are provided onto the surface of the solid carrier by the following steps in the order:
(a) providing a first mesh (3) on the surface of the solid carrier comprising the plurality of spots having the spot size X, wherein the first mesh has a mesh size M1 of n·0.8X ≤ M1 ≤ n·1.2X, wherein n is a natural number of at least 1;
(b) providing a second mesh (4) on the first mesh, wherein the second mesh has a mesh size M2 which is smaller than the mesh size M1 of the first mesh;
(c) providing cells onto the second mesh and thereby bringing the cells into contact with the surface of the solid carrier; and
(d) removing the first mesh and the second mesh.

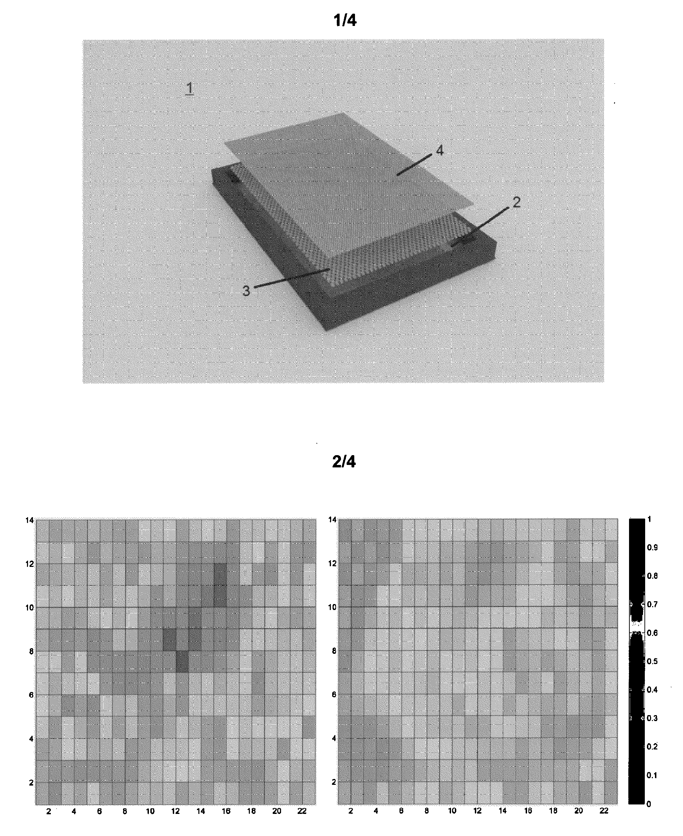

Figure 1 shows a preferred embodiment of the cell array (1) comprising a solid carrier (2), a first mesh (3) provided on the surface of the solid carrier and a second mesh (4) provided on the first mesh.

In the following, the terms "cell array" and "cell chip" will be used synonymously.

The term "solid carrier" as used herein does not have any specific limitations, and relates, for example, to glass or an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextrane, agarose, chitin and polyamino acids, or an inorganic polymer, such as glass or metallohydroxide. The solid carrier can be in the form of a microcarrier, particles, membranes, strips, paper, film, or plates, such as microtiter plates or microarrays. The term "microarray" as used herein may mean any arrangement of the spots in addressable locations on a support resulting in a so-called "biochip".

The length and width of the solid carrier are not particularly limited as long as the dimensions of the solid carrier are compatible with a data acquisition device (e.g. a microscope). According to a preferred embodiment of the present invention, the length is from 70 mm to 180 mm, preferably from 80 mm to 160 mm, more preferably from 90 mm to 140 mm and most preferably from 100 mm to 120 mm. The width of the solid carrier is preferably from 40 mm to 100 mm, more preferably from 50 mm to 90 mm and most preferably from 60 mm to 80 mm. In case a high-density cell array is used, the length is preferably 109 mm and the width 75 mm.

Preferably, the solid carrier is a coated solid carrier, for example with collagen, fibronectine, GAPS (Gamma Amino Propyl Silane), sucrose, Silane, Matrigel or polylysine or pretreated with diluted NaOH.

Preferably, the solid carrier is coated with a cell-repelling agent such as flat hydrophilic surfaces, coated with a cell-adhesive agent such as collagen or fibronectin, or uncoated.

The term "cell-repelling" used herein means a state of inhibiting adhesion of a cell. The term "cell-adhesive" according to the present invention means a state of enabling adhesion of a cell.

Preferably, the solid carrier is a cell culture device.

According to the present invention, the term "spot" refers to an area on the surface of the solid carrier. The spot may comprise reagents which may be brought at the spot on the surface of the solid carrier by various methods. A contact printing method including spotting with a needle, for example having a diameter of 150 to 600 µm, a contactless method such as for example a piezoelectric printer, application of molecular self-assembly, printing with an atomic force microscope or a combination of different methods may be used to bring the reagents at the spot on the surface of the solid carrier.

Reagents comprised in the spot are not particularly limited. Preferably, each of the plurality of spots comprises a transfection mixture comprising a transfection agent, at least one protein, preferably a polypeptide or an antibody, and/or one nucleic acid molecule, which may have any kind of modification (e.g. attached phosphor and methyl groups).

The transfection reagent may be any commercially available transfection agent which is suitable for transfecting proteins into cells. The term "transfection agent" is not limited to agents which are suitable for transfecting proteins and/or nucleic acid molecules into cells, but also comprises agents which are suitable to transfect other molecules into cells, like for example DNA, RNA, siRNA, proteins or antibodies. Any transfection agent, which does not prevent the transfection of proteins and/or nucleic acid molecules into cells, is a transfection agent according to the term "transfection agent" as used herein. In a preferred embodiment, the transfection agent is selected from the group consisting of Novagen ProteoJuice, OZ Biosciences DreamFect Gold, ibidi Torpedo DNA, Peqlab PeqFect siRNA, Invitrogen Lipofectamine 2000, Invitrogen Lipofectamine RNAiMAX and Gelantis GeneSilencer.

The term "protein" used herein does not underlie a specific restriction and may include any protein, protein complex or polypeptide, including recombinant proteins, protein complexes and polypeptides either isolated from a natural source or obtained via recombinant DNA technology, or a biologically active derivative thereof.

As used herein, the term "biologically active derivative of a protein" includes any derivative of a protein, protein complex or polypeptide having substantially the same functional and/or biological properties of the protein such as binding properties, and/or the same structural basis, such as a peptidic backbone. The polypeptide sequences of the functionally active derivatives may contain deletions, additions and/or substitution of amino acids whose absence, presence and/or substitution, respectively, do not have any substantial negative impact on the activity of the polypeptide, e.g. amino acids which are located in a part of the polypeptide sequence that does not contribute to the biological activity of the protein. Minor deletions, additions and/or substitutions of amino acids of the respective polypeptide sequences which are not altering the biological activity of said polypeptide are also included in the present application as biologically active derivatives.

A protein obtained from a natural source may be any protein isolated from a natural source, like for example a body fluid derived from a mammal. In a preferred embodiment, the mammal is selected from the group consisting of mouse, human, rat, cat, dog, and monkey.

The protein may be produced by any method known in the art. This may include any method known in the art for the production of recombinant DNA by genetic engineering, e.g. via reverse transcription of RNA and/or amplification of DNA. This includes methods which comprise the recombinant production of the protein.

The recombinant protein used may be produced by any method known in the art. This may include any method known in the art for (i) the production of recombinant DNA by genetic engineering, e.g. via reverse transcription of RNA and/or amplification of DNA, (ii) the introduction of recombinant DNA into prokaryotic or eukaryotic cells by transfection, e.g. via electroporation or microinjection, (iii) the cultivation of said transformed cells, e.g. in a continuous or batchwise manner, (iv) the expression of the protein, e.g. constitutive or upon induction, and (v) the isolation of the protein, e.g. from the culture medium or by harvesting the transformed cells, in order to (vi) obtain purified recombinant protein, e.g. via anion exchange chromatography or affinity chromatography.

For example, the recombinant DNA coding for the protein, e.g. a plasmid, may also contain a DNA sequence encoding a selectable marker for selecting the cells which have been successfully trarisfected with the plasmid. In one example, the plasmid may also confer resistance to a selectable marker, e.g. to the antibiotic drug G418, by delivering a resistance gene, e.g. the neo resistance gene conferring resistance to G418.

The production of the protein may include any method known in the art for the introduction of recombinant DNA into cells, which might be mammalian cells, bacterial cells or insect cells, by transfection, e.g. via electroporation or microinjection. For example, the recombinant expression of the protein can be achieved by introducing an expression plasmid containing the human the protein encoding DNA sequence under the control of one or more regulating sequences such as a strong promoter, into a suitable host cell line by an appropriate transfection method resulting in cells having the introduced sequences stably integrated into the genome. The calcium-phosphate co-precipitation method is an example of a transfection method.

The production of the protein may also include any method known in the art for the cultivation of said transformed cells, e.g. in a continuous or batchwise manner, and the expression of the protein e.g. constitutive or upon induction. In one specific example, the nucleic acid coding for the protein contained in a host organism is expressed via an expression mode selected from the group consisting of induced, transient, and permanent expression. Any expression system known in the art or commercially available can be employed for the expression of a recombinant nucleic acid encoding the protein, including the use of regulatory systems such as suitable, e.g. controllable, promoters, enhancers etc.

The production of the protein may also include any method known in the art for the isolation of the protein, e.g. from the culture medium or by harvesting the transformed cells. For example, the protein-producing cells can be identified by isolating single-cell derived populations i.e. cell clones, via dilution after transfection and optionally via addition of a selective drug to the medium. After isolation, the identified cell clones may be cultivated until confluency in order to enable the measurement of the protein content of the cell culture supernatant by enzyme-linked immuno-sorbent assay (ELISA) technique.

Additionally, the production of the protein may include any method known in the art for the purification of the protein, e.g. via anion exchange chromatography or affinity chromatography. In one preferred embodiment the protein can be purified from cell culture supernatants by semi-affinity calcium-dependent anion exchange chromatography, e.g. in an endotoxin-free system. The purified protein may be analyzed by methods known in the art for analyzing recombinant proteins, e.g. the ELISA technique. In addition, the protein integrity and activity may be assessed. It is within the knowledge of a person skilled in the art to choose the optimal parameters, such as buffer system, temperature and pH for the respective detection system to be used.

In one specific example, the protein transfected into a cell is expressed in a host cell type with the ability to perform posttranslational modifications. The ability to perform posttranslational modifications of the protein expressing host cell lines may be for example analyzed by mass-spectrometric analysis.

The host cell type used for the recombinant production of the protein may be any bacterial or mammalian cell, preferably with the ability to perform posttranslational modifications of the protein. There is no particular limitation to the media, reagents and conditions used for culturing the cells in the cell culture used for the recombinant protein production of including culturing the cells in a continuous or batchwise manner. The desired protein which has been expressed by the cells of the and which, dependent on the transfection/vector-system used, is contained in the cells or secreted into the medium for culturing cells, can be isolated/recovered from cell culture using methods known in the art.

In a preferred embodiment, the protein is an antibody or a fragment thereof.

The term "antibody" as used herein may be any antibody including biologically active fragments of an antibody having substantially the same biological function as the antibody, e.g. the ability to bind a specific antigen. In particular, the antibody can be selected from the group consisting of a naturally occurring antibody, a polyclonal antibody, a chimeric antibody, a monoclonal antibody, e.g. derived by conventional hybridoma techniques, and an antibody or antibody fragment obtained by recombinant techniques, e.g. phage display or ribosome display, mutated antibodies and (semi)-synthetic antibodies. For example, the antibody or at least one fragment thereof may contain one or more mutations or variations, such as added, deleted or substituted amino acids or nucleic acids, as long as it has no negative effect on the interaction with the specific antigen. The antibody may belong to any immunoglobulin class.

The term "fragment" used herein means any portion of an antibody as defined above as long as it has the ability to bind to the desired antigen. Moreover, a fragment may comprise several different portions from said antigen. Examples of fragments of an antibody antigen binding fragment (FAB), single chain variable fragment (scFv), variable domain of the heavy chain (VH), variable domain of the light chain (VL), complementary determining regions (CDRs), and combinations thereof. The term "scFv" used herein means a fusion of the variable regions of the heavy and light chains of any immunoglobulin, linked together with a linker, such as for example serine, glycine, any other natural or non-natural amino acid, a peptide, a protein, or a nucleic acid.

For example, the antibody or the at least one fragment thereof may be a humanized antibody or at least one fragment thereof derived from the murine antibody H398. There is no limitation as to the technique of humanization of the antibody, as long as the antibody binds to the desired antigen. Examples of humanization include CDR-grafting, The expression "humanized antibody" used herein means any antibody in which protein engineering is used to reduce the amount of foreign ("non-human") protein sequence by swapping e.g. rodent antibody constant regions and/or the variable-domain framework with sequences that are found in human antibodies. In a further embodiment of the antibody fragment as defined above, the at least one fragment is a Fab-region, a scFv, or any post-translationally processed recombinant derivative thereof.

In another preferred embodiment, the protein is a nuclease being involved in genome editing, like for example CAS9, transcription-like effector (TALEN), or zinc-finger nucleases.

The term "nucleic acid molecule" used herein does not underlie a specific restriction and may include any nucleic acid molecule of any length, including polynucleotides, oligonucleotides, aptamers, deoxyribonucleic acid (DNA), ribonucleic acid (RNA), and artificial nucleic acid analogs, like morpholinos, bridged nucleic acid (BNA), peptide nucleic acid (PNA), locked nucleic acid (LNA), glycol nuclei acid (GNA), and therose nucleic acid (TNA). In a preferred embodiment the at least one nucleic acid molecule is selected from the group consisting of DNA, mRNA, siRNA, miRNA, sgRNA, gRNA, cDNA, DNA aptameres, viral transfection vectors, and morpholino oligomers.

The siRNA molecule can be any short double stranded RNA, preferably of 15 to 30 nucleotides, more preferably of 19 to 25, most preferably of 21 to 23 nucleotides length. The siRNA may contain modifications within the double-stranded region, including modifications of nucleobases and/or modifications of the backbone, as well as overhangs at the 3' and/or 5' end of the sense and/or antisense strand.

The cDNA molecule can be any cDNA molecule known in the art. Methods for obtaining cDNA by synthezing DNA using a messenger RNA (mRNA) as a template employing the reverse transciptase function of a DNA polymerase are known to a person skilled in the art. In a preferred embodiment, the cDNA molecule is a double-stranded DNA molecule having a length form 20 bp to 15000 bp, more preferably from 40 bp to 6000 bp, more preferably from 100 bp to 4000 bp.

The term nucleic acid molecule as used herein includes recombinant and synthetic nucleic acid molecules as well as nucleic acid molecules which are isolated from a natural source, including biologically active derivatives thereof.

As used herein, the term "biologically active derivative of a nucleic acid molecule" includes any derivative of a nucleic acid molecule having substantially the same functional and/or biological properties of the at least one nucleic acid molecule such as coding properties, and/or the same structural basis, such as a nucleotide backbone. The nucleotide sequences of the functionally active derivatives may contain deletions, additions and/or substitution of nucleotides whose absence, presence and/or substitution, respectively, do not have any substantial negative impact on the activity of the nucleic acid molecule, e.g. nucleotides which are located in a part of the nucleotide sequence that does not contribute to the biological activity of the nucleic acid molecule. Minor deletions, additions and/or substitutions of nucleotides of the respective nucleotide sequences which are not altering the biological activity of said nucleic acid molecule are also included in the present application as biologically active derivatives.

A nucleic acid molecule obtained from a natural source may be any nucleic acid molecule isolated from a natural source, like for example a tissue or cell sample derived from a mammal. In a preferred embodiment, the mammal is selected from the group consisting of mouse, human, rat, cat, dog, and monkey.

The nucleic acid molecule may be produced by any method known in the art. This may include any method known in the art for the production of recombinant DNA by genetic engineering, e.g. via reverse transcription of RNA and/or amplification of DNA. This includes methods which comprise the recombinant production of the nucleic acid molecule.

The recombinant nucleic acid molecule used may be produced by any method known in the art. This may include any method known in the art for (i) the production of recombinant DNA by genetic engineering, e.g. via reverse transcription of RNA and/or amplification of DNA, (ii) the introduction of recombinant DNA into prokaryotic or eukaryotic cells by transfection, e.g. via electroporation or microinjection, (iii) the cultivation of said transformed cells, e.g. in a continuous or batchwise manner, and (iv) the isolation of the nucleic acid molecule, e.g. by harvesting the transformed cells, in order to (v) obtain purified recombinant nucleic acid molecule, e.g. via anion exchange chromatography or affinity chromatography.

For example, the recombinant nucleic acid molecule, e.g. a plasmid, may also contain a DNA sequence encoding a selectable marker for selecting the cells which have been successfully transfected with the plasmid. In an example, the plasmid may also confer resistance to a selectable marker, e.g. to the antibiotic drug G418, by delivering a resistance gene, e.g. the neo resistance gene conferring resistance to G418.

The production of the nucleic acid molecule may include any method known in the art for the introduction of recombinant DNA into cells, which might be mammalian cells, bacterial cells or insect cells, by transfection, e.g. via electroporation or microinjection. The production of the nucleic acid molecule may also include any method known in the art for the cultivation of said transformed cells, e.g. in a continuous or batchwise manner.

The production of the nucleic acid molecule may also include any method known in the art for the isolation of cells containing the nucleic acid molecule, e.g. from the culture medium or by harvesting the transformed cells. For example, the nucleic acid molecule containing cells can be identified by isolating single-cell derived populations i.e. cell clones, via dilution after transfection and optionally via addition of a selective drug to the medium. After isolation, the identified cell clones may be cultivated until confluency.

Protocols for recombinant DNA isolation, cloning and sequencing are well known in the art. Further, methods for the production and isolation of RNA molecules are well known in the art.

According to the present invention, the solid carrier contains 9 to 400 spots per cm², more preferably 100 to 400 spots per cm², even more preferably 150 to 400 spots per cm², and most preferably 200 to 400 spots per cm². In case a high-density cell array is used, the spot density preferably amounts to 275 to 295 spots per cm².

The spot size on the surface of the solid carrier does not have any specific limitations. In a preferred embodiment according to the present invention, each spot has a spot size of 5 to 750 µm in diameter, more preferably of 100 to 600 µm in diameter, even more preferred of 200 to 500 µm in diameter and most preferably of 250 to 400 µm in diameter. In case a high-density cell array is used, the spot size is preferably from 250 to 300 µm in diameter. The spot size can be evaluated using e.g. transmission light microscopy, fluorescence microscopy or other surface characterization methods such as surface plasmon resonance (SPR).

The term "plurality" according to the present invention means more than one.

In a preferred embodiment of the present invention, the cells on the surface of the solid carrier have a substantially uniform distribution. The evaluation method of a uniform cell distribution according to the present invention is not particularly limited and may be performed by counting the cells. The number of cells on the surface of the solid carrier may be measured by any method known in the art for counting cells. This may include hemocytometers, automated cell counters, Coulter counters, spectrophotometry and plating.

An example of an evaluation method of a uniform cell distribution is shown in Figure 2. Figure 2 shows a cell distribution on a surface of a solid carrier of a cell array, wherein the cells have been provided without the use of a mesh on the left and of a cell array according to the present invention on the right. The cells distributed on the surface of the solid carrier are imaged. The imaging is not particularly limited and may be carried out by any commonly used technique, for example by staining the nuclei with bisbenzimide (Hoechst 33342). The image of the cell distribution on the surface is segmented into segments sᵢ and the number of cells for each segment sᵢ is calculated by any of the above methods for counting cells. In Figure 2, each segment sᵢ is represented by a rectangle. The segment with the highest amount of cells (sₘₐₓ) is identified. The degree of uniformity of the cell distribution on the whole surface of the solid carrier is evaluated by calculating the ratio of the number of cells in each segment sᵢ to the number of cells in sₘₐₓ, i.e. sᵢ/sₘₐₓ. The ratio approaches 0 for a segment with a small number of cells and 1 for a segment with a high number of cells similar to segment sₘₐₓ. According to a preferred embodiment of the present invention, the cells on the surface of the solid carrier have a substantially uniform distribution such that more than 60% of the segments have a ratio of 0.3 ≤ sᵢ/sₘₐₓ ≤ 0.7, further preferred more than 70% of the segments have a ratio of 0.3 ≤ sᵢ/sₘₐₓ ≤ 0.7, even more preferred more than 80% of the segments have a ratio of 0.3 ≤ sᵢ/sₘₐₓ ≤ 0.7 and most preferred more than 90% of the segments have a ratio of 0.3 ≤ sᵢ/sₘₐₓ ≤ 0.7.

Within the scope of the invention, the term "cells" means a generic term and encompass the cultivation of individual cells, tissues, organs, insect cells, avian cells, mammalian cells, bacterial cells, primary cells, continuous cell lines, stem cells and/or genetically engineered cells, such as recombinant cells expressing a hetereologous polypeptide or protein and/or containing a recombinant nucleic acid molecule and/or stable expressing cells.

Preferably, the cell is a cell derived from a cell culture or a cell derived from a sample taken from an individual.

The term "individual" as used herein is not limited to any particular individual and may be a fungus, an animal, a human being or a plant.

The sample taken from an individual may be any sample. Preferably, the sample may be derived from a healthy or diseased naturally occurring system, preferably a sample containing a body fluid or components derived from a body fluid. Further, the sample may be a naturally occurring system such as a solution selected from the group consisting of serum, saliva, urine, bile, lymph, tissue, like e.g. bladder or kidney, cerebrospinal fluid and/or other body fluids in the case of an animal or human individual.

Furthermore, the sample may be derived from a healthy or diseased naturally occurring system, preferably a sample containing a tissue or components derived from a tissue. A tissue or a component derived therefrom may be of various origins, like for example leafs, roots, petals, germs or stems in the case of plant individual, or muscle tissue, heart tissue, liver tissue, kidney tissue, epithelial tissue, connective tissue, or nervous tissue.

The term "cell culture" in its various grammatical forms, refers to cells grown in suspension, roller bottles, flasks and the like. Large scale approaches, such as bioreactors, including adherent cells growing attached to microcarriers in stirred fermentors, also are included. Moreover, it is possible to not only to culture surface-dependent cells, but also to use the suspension culture techniques. If the cells are grown on microcarriers, the microcarrier can be selected from the group of microcarriers based on dextran, collagen, plastic, gelatin, membranes in general and cellulose and others.

The cells derived from a cell culture may be derived from animals, plants or fungus. Animal cells may be derived for example from insects, fish, birds, or mammals. Preferably, cells derived form a cell culture are mammalian cells including those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells that can cultivated in the culture medium can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, 293 cells, NS-1 cells MRC-5 cells, WI-38 cells, BHK cells, 293 cells, HeLa, MCF7, MCF10a, RK-cells and stem cells (e.g. iPS).

The sample may be derived from a mammal, preferably a mammal selected from the group consisting of human, mouse, rat, pig, cat, dog, horse, goat, cattle, cow, and monkey and/or others. Preferably, the sample is derived from a human. Further, the sample may contain isolated body fluid compounds or processed body fluids derived from a mammal, preferably a mammal selected from the group consisting of human, mouse, rat, pig, cat, dog, horse, goat, cattle, cow, and monkey and/or others. Preferably, the sample contains isolated body fluid compounds or processed body fluids derived from a human.

The first mesh and the second mesh according to the present invention are not specifically limited as long as the first mesh has the mesh size M1 and the second mesh has the mesh size M2. In a preferred embodiment of the present invention, the mesh size M1 of the first mesh is in the range of n·0.8X ≤ M1 ≤ n·1.2X, preferably in the range of n·0.9X ≤ M1 ≤ n·1.1X, more preferably in the range of n·0.95X ≤ M1 ≤ n·1.05X, wherein the factor n is a natural number of at least 1. According to an embodiment of the present invention, n may be a natural number from 1 to 4, preferably from 1 to 3, more preferably 1 or 2 and, most preferably, n is 1. Further, according to a preferred embodiment, the mesh size M2 of the second mesh is smaller than the mesh size M1 of the first mesh. In a preferred embodiment of the present invention, the mesh size M2 is ≤ 0.5M1, more preferably M2 is ≤ 0.45M1 and most preferably M2 is ≤ 0.4M1. Preferably, the mesh size M1 of the first mesh is from 300 to 700 µm, more preferably from 400 to 600 µm, most preferably from 450 to 550 µm. Further, the mesh size M2 of the second mesh is preferably from 50 to 300 µm, more preferably from 75 to 250 µm, most preferably from 100 to 200 µm.

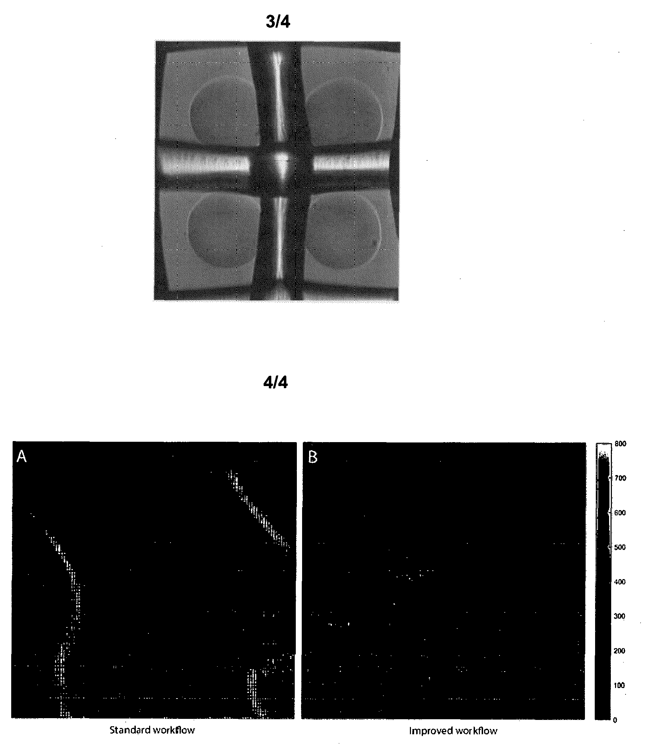

By providing the first mesh and the second mesh on the surface of the solid carrier, temporary wells (floating wells) are created on the spots on the surface of the solid carrier as shown in for example Figure 3, wherein the first mesh acts as a temporary border to the cells and the transfection material. The first mesh and the second mesh used in the present invention filter the cell suspension which is provided onto the second mesh and segregates the total volume of the suspension into smaller volumes, thereby making the material (i.e. the cells and the substances contained in the spots) present in the floating wells on the surface of the solid carrier less feasible to mix. Therefore, the first mesh and the second mesh block cell and transfection material transport by providing temporary borders and enable a homogeneous cell and transfection material distribution and, furthermore, avoid sample crosstalk and cross contamination. Crosstalk is an undesired sample leakage from one separate probe to another separate probe.

The advantageous homogeneous distribution of the material, particularly of the cells, is indicated in for example Figure 2. Figure 2 shows a cell distribution on a surface of a solid carrier of a cell array, wherein the cells have been provided without the use of a mesh on the left and of a cell array according to the present invention on the right. The cell distribution on the cell array without the application of a mesh is clearly inhomogeneous as indicated by the dark color in the centre and at the border of the graph on the left of Figure 2. The presence of cells at the border is caused by the coffee ring effect while e.g. the weight of the media on top of the solid carrier leads to bending of said carrier, thereby promoting an accumulation of cells in the centre of the solid carrier. The coffee ring effect describes particle transport, in the present case in particular cell and transfection material transport, outwards from a centre of a volume due to flow currents generated by evaporation of a solution at the border of the volume which results in an inhomogeneous cell and transfection material distribution. On the contrary, the graph on the right side of Figure 2 displays only mostly areas of light grey colors which represent a homogeneous cell distribution.

Further, Figure 4 shows evaluated data resulting from the application of fluorescence microscopy and a cell counting method of a cell distribution on a surface of a solid carrier of a cell array, wherein the cells have been provided without the use of a mesh on the left (standard workflow) and of a cell array according to the present invention on the right (improved workflow). In Figure 4, the cell number in a field of view (sample spot) is characterized by the scale on the right of said Figure 4. A dark color represents a small amount of cells and a bright color a high amount of cells. Referring to the right side of Figure 4, mostly dark colors are present while the left side of Figure 4 shows several areas of bright colors. Therefore, the cell array according to the present invention provides a more homogeneous cell distribution compared to the cell array without inserted meshes.

Accordingly, the cell array according to the present invention combines the advantages of multiwell plates, like reduced material flow, and of microarrays, like parallel processing in a single assembly, by providing the temporary wells (floating wells).

The material of the first mesh and the second mesh is not specifically limited. In a preferred embodiment of the present invention, the material of the first mesh and/or the second mesh is a cell-repelling or a cell inert material.

The term "cell inert material" used herein means a material not substantially interacting with cells.

When using such a cell-repelling or cell inert material, the cells are contained in spots without any mesh-cell interaction effects. On the other hand, the use of cell-adhesive materials for the mesh could be advantageous in 3D cell culture. The material of the first mesh and/or second mesh may be coated with a coating agent. The coating agent is not particularly limited and depends on the material used for the first and the second mesh. Avidin-biotin interaction could for example be used to coat the mesh. Preferably, the surface of the first mesh and/or the second mesh comprises or consists of a cell-repelling or cell inert material. In case that a cell-adhesive material is used as the material of the first mesh and/or the second mesh, the surface of the first mesh and/or the second mesh comprises or consists of a cell-repelling or cell inert material which may be applied to the first mesh and/or second mesh by coating.

The cell-repelling material is not specifically limited and can be any material as long as it inhibits adhesion of a cell to the material. In particular, commonly known polymers and copolymers may be used as cell-repelling material. The cell-repelling material may be for example polystyrene, polyamides, polycarbonate or poly(methyl methacrylate). Preferably, polyamides, more preferably nylon, is used as a cell-repelling material.

As the first mesh and/or the second mesh a commercially available mesh may be used like for example a mesh from Fisher. An example of the first mesh may be MEMBRAN NYLON 1M X 1M 500 µM, item no.: 11755498, Fisherbrand, order number: 1000X1000M500UM and for the second mesh may be MEMBRAN NYLON 1M X 1M 150 µM, item no.: 11705498, Fisherbrand, order number: 1000X1000M150UM. Such meshes may be modified for example by coating it with a cell-repellent or cell inert material.

Preferably, the first mesh and the second mesh are reusable.

Further, the present invention relates to a manufacturing method of a cell array, wherein the method comprises the following steps in the order:
(a) providing a first mesh on a surface of a solid carrier containing a plurality of spots having a spot size X, wherein the first mesh has a mesh size M1 of n·0.8X ≤ M1 ≤ n·1.2X, wherein n is a natural number of at least 1;
(b) providing a second mesh on the first mesh, wherein the second mesh has a mesh size M2 which is smaller than the mesh size M1 of the first mesh;
(c) providing cells onto the second mesh and thereby bringing the cells into contact with the surface of the solid carrier; and
(d) removing the first mesh and the second mesh.

The terms "solid carrier", the "spot" , the "cells" and the first mesh and the second mesh of the manufacturing method according to the present invention correspond to the above definitions of said terms for the cell array according to the present invention.

In step (d), the first mesh and the second mesh may be removed as soon as the cells adhere onto the surface of the solid carrier. Preferably, step (d) is carried out 3 to 5 hours, more preferably 4 hours, after step (c).

The present invention also relates to the use of the above-defined cell array for solid phase transfection, wherein each of the plurality of spots comprises a transfection mixture comprising a transfection agent, at least one protein and/or at least one nucleic acid molecule, and the spots are brought into contact with the cells on the surface of the solid carrier.

Several ways of solid phase transfection can be used according to the present invention.

Preferably, the transfection mixture comprising a transfection reagent, at least one protein and/or at least one nucleic acid molecule is spotted on at least part of the solid carrier, for example with a small metal pin, prior to step (a) of the provision of cells on the above cell array such that each of the plurality of spots comprises a transfection reagent, at least one protein and/or at least one nucleic acid molecule. This procedure can be followed by a drying step to provide longer storage by a longer stability of the transfection complexes, for example by freeze-drying. After the cell seeding only the cells which grow on the spotted regions take up the complexes, cells in other regions do not take up the complexes. This procedure has the advantage that more and/or different transfection approaches can be prepared on one solid carrier, because less space is needed. For example one transfection approach on a 400 µm-spot covers 1.26 x10⁻⁷ m².

In a preferred embodiment, the transfection mixture is mixed together in each of the plurality of spots of the solid carrier. In another preferred embodiment, the transfection mixture is mixed together before being applied to each of the plurality of spots of the solid carrier. In a particularly preferred embodiment, the transfection mixture is mixed together before being applied to each of the plurality of spots of the solid carrier and a complex of the transfection agent with the at least one protein and/or the at least one nucleic acid molecule is formed before the complex is applied to each of the plurality of spots of the solid carrier.

In a preferred embodiment of the method of solid phase transfection, each of the plurality of spots comprising a transfection reagent, at least one protein and/or at least one nucleic acid molecule on the solid carrier further contains a sugar, preferably a non-reducing disaccharide, preferably trehalose or sucrose. Thus, in a preferred embodiment the transfection mixture in the spot comprising a transfection reagent, at least one protein and/or at least one nucleic acid molecule on the solid carrier further contains a sugar, preferably sucrose. The sugar may be present in a fluid in any suitable concentration, preferably 0.2 to 0.8 M, more preferably 0.3 to 0.6 M, most preferably 0.4 M.

Further, in another preferred embodiment the transfection mixture in each of the plurality of spots comprising a transfection reagent, at least one protein and/or at least one nucleic acid molecule on the solid carrier further contains a cell culture medium, preferably, a low serum cell culture medium, most preferably OptiMEM produced by Invitrogen. In another preferred embodiment, the cell culture medium is a serum-free medium, preferably a protein-free medium.

In a more preferred embodiment, the transfection mixture in each of the plurality of spots comprising a transfection reagent, at least one protein and/or at least one nucleic acid molecule on the solid carrier further contains a sugar as defined above being dissolved in a cell culture medium as defined above. In a most preferred embodiment, the spot contains a solution of 0.4 M sucrose in a low serum cell culture medium, most preferably OptiMEM produced by Invitrogen.

In one embodiment, the transfection mixture in each of the plurality of spots comprising a transfection reagent, at least one protein and/or at least one nucleic acid molecule on the solid carrier and optionally the sucrose and/or cell culture medium are incubated after they have been applied to the spot on the solid carrier. Therefore, in a preferred embodiment, the method for solid phase transfection contains after the spotting step and before bringing the cells into contact with the spots an additional step of incubating the transfection reagent and the at least one protein and/or the at least one nucleic acid molecule and optionally the sucrose and/or cell culture medium comprised in each of the plurality of spots for at least 1 minute, preferably, 2 to 360 minutes, more preferably 5 to 60 minutes, more preferably 10 to 40 minutes, most preferably 15 to 30 minutes. In a preferred embodiment, the incubation is carried out between 4°C to 40°C, preferably between 15°C and 37°C, more preferably between 17°C and 27°C, and most preferably at room temperature.

The incubation step may further contain the incubation of the transfection reagent and the at least one protein and/or the at least one nucleic acid molecule with sucrose and/or cell culture medium as defined above.

In a preferred embodiment, the transfection mixture comprised in each spot comprising a transfection reagent, at least one protein and/or at least one nucleic acid molecule on the solid carrier further contains a glycoprotein, preferably a high-molecular weight glycoprotein, most preferably fibronectin. In a preferred embodiment, the glycoprotein is mixed with gelatin. In a preferred embodiment, the mixture contains 0.1 % to 5%, more preferably 0.2 to 3%, more preferably 0.5 to 1.5% and most preferably 1% glycoprotein. In a preferred embodiment, the mixture contains 0.005% to 2%, more preferably, 0.05 % to 1 %, more preferably 0.1 to 0.5 %, and most preferably 0.2 % gelatin.

In a preferred embodiment, the method of solid phase transfection contains after the step of spotting and before the step of bringing the cells into contact with the spots a step wherein the mixture of the glycoprotein and the gelatin as defined above is added to the transfection mixture in a further step.

In a most preferred embodiment, the method of solid phase transfection comprises the two additional steps, i.e. the incubation step and the step of adding a glycoprotein or a mixture of a glycoprotein and gelatin to the spot, in that order.

In the step of bringing the cells into contact with the plurality of spots wherein each spot comprises a transfection mixture comprising a transfection reagent, at least one protein and/or at least one nucleic acid molecule on the solid carrier, the transfection of the at least one protein and/or the at least one nucleic acid molecule in each spot of the plurality of spots into the cells is carried out. It is within the knowledge of the person skilled in the art to choose the optimal parameters, such as buffer system, temperature and pH for the respective fluid to be used for this step.

The transfection mixture in the spot comprising a transfection reagent, at least one protein and/or at least one nucleic acid molecule on the solid carrier may further contain any salts or buffer agents which do not prevent the transfection of the protein and/or the nucleic acid molecule into the cell.

In a preferred embodiment, the cells are incubated after being brought into contact with the plurality of spots. The incubation might be of use for the transfection of the protein and/or the nucleic acid molecule into the cell. In a preferred embodiment, the incubation is carried out between 5°C to 40°C, preferably between 15°C and 37°C, more preferably between 17°C and 27°C, and most preferably at room temperature. In another preferred embodiment the cells are incubated at 37°C, preferably at an atmosphere containing 5% CO₂.

In a preferred embodiment, the cells are incubated for at least 0.5, 1, 2, 3, 4, 6, 8, 10, 12, 18, 24, 36, 48, or 72 hours. In a most preferred embodiment, the incubation is carried out from 3 to 72 hours.

According to a preferred embodiment, the protein and/or the nucleic acid molecule is covalently linked to at least one lipid moiety.

According to another preferred embodiment, the protein and/or the nucleic acid molecule is covalently linked to at least one detectable substance. The detectable substance may be used for detecting the protein and/or the nucleic acid molecule in the cell and/or outside of the cell after transfection. The detection in the cell might be carried out inside the intact cell or after cell lysis. The detection outside of the cell might be carried out at the cell surface or in the liquid being in contact with the cell.

The term "detectable substance" does not exhibit any particular limitation and may be selected from the group consisting of radioactive labels, fluorescent dyes, compounds having an enzymatic activity, magnetic labels, antigens, and compounds having a high binding affinity for a detectable substance. A compound having an enzymatic reactivity such as the enzyme luciferase which produces a light signal upon contact with the respective substrate can also be used as a detectable substance which may be linked covalently to said substrate. Coupling a detectable substance to an antigen allows the detection of the substance by an antibody/enzyme-complex (the enzyme being e.g. phosphatase) catalysing a detectable color reaction when using a suitable substrate. A compound with a high binding affinity for a different detectable substance such as biotin which binds to a detectable substance covalently linked to e.g. streptavidin, is a further possibility for making a substance detectable. In a preferred embodiment, the detectable substance is a substance which can be detected inside of a cell. In a preferred embodiment, the detectable substance is a fluorescent dye.

In one embodiment, the protein and/or the nucleic acid molecule is linked to a detectable substance and the method for solid phase transfection further comprises a step of detecting the detectable substance after bringing the cells into contact with the plurality of spots.

The reaction conditions for detecting the detectable substance depend upon the detection method selected. It is within the knowledge of the person skilled in the art to choose the optimal parameters, such as buffer system, temperature and pH for the respective detection system to be used.

The detection of the detectable substance may comprise one or more detection method(s) selected from the group consisting of immunoblotting, immunoprecipitation, immunocapture, monoclonal antibody immobilization of platelet antigens or enzyme linked immuno sorbent assay (ELISA), flow cytometry, protein array technology, spectroscopy, mass spectrometry, chromatography, surface plasmonic resonance, fluorescence extinction and/or fluorescence energy transfer. The detection method for measuring the detectable substance can, for example, be selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of the person skilled in the art to choose the optimal parameters, such as buffer system, temperature and pH for the respective detection system to be used.

In a preferred embodiment, at least part of the transfection mixture containing the transfection reagent and the at least one protein and/or the at least one nucleic acid molecule comprised in each spot are dried or frozen. In a preferred embodiment the transfection mixture containing the transfection reagent and the at least one protein and/or the at least one nucleic acid molecule comprised in each spot are dried or frozen. In a preferred embodiment, the cells to be transfected are seeded straight on the frozen or dried transfection mixture. In another preferred embodiment, the cells to be transfected are seeded on the de-frozen or re-hydrated transfection mixture.

Methods for drying reactants on a solid surface are known in the art. Any suitable method known in the art for drying a reactant to a solid surface, preferably without adding any further component to the reactant, can be used. A dried spot is preferably obtained (i) by vacuum centrifugation followed by lyophilisation or (ii) by freeze drying, steps (i) or (ii) are preferably carried out after the spot has been frozen to -20°C. In a preferred embodiment, a frozen spot is obtained by freezing the solid carrier to at least 0°C, at least -5°C, at least -10°C, at least -15°C, at least -20°C, at least -30°C, or at least -80°C.

In a preferred embodiment, the sample is pre-purified before bringing the cells into contact with the spots of the method of the solid phase transfection. In a more preferred embodiment, the pre-purification comprises the step of removing impurities that impair significantly or prevent the transfection of the protein into the cell.

According to the present invention, by providing the first mesh and the second mesh on the surface of the solid carrier, temporary borders are created on the spots on the surface of the solid carrier which can block cell and transfection material transport and thereby enable a homogeneous cell and transfection material distribution and, furthermore, avoid sample crosstalk and cross contamination. Therefore, the cell array according to the present invention combines the advantages of multiwell plates, like reduced material flow, and of microarrays, like parallel processing in a single assembly, by providing the temporary wells (floating wells).These advantageous effects are in particular beneficial in cell arrays and high-density cell arrays (HD-CA), since in said devices physical borders between spots are not present. Therefore, the present invention enables the advantageous use of cell arrays and, in particular, HD-CA having a high number of spots of up to 24576, without having the problems of an inhomogeneous cell and transfection material distribution, sample crosstalk and cross contamination. Accordingly, the present invention enables for example an efficient performance of genome wide cell-based screening in just one day, functional assessment of large classes of molecules, such as ever growing numbers of non-coding RNAs (ncRNAs), and combinatorial genetic or chemical screening which is an awaited technology for efficient drug discovery.

The figures show:
Figure 1: A preferred embodiment of the cell array (1) according to the present invention comprising a solid carrier (2), a first mesh (3) on the surface of the solid carrier and a second mesh (4) on the first mesh.
Figure 2: An example of cell distribution on a surface of a solid carrier of a cell array, wherein the cells have been provided without the use of a mesh on the left and of a cell array according to the present invention on the right.
Figure 3: An example of a mesh provided on a surface of a solid carrier, thereby creating temporary borders (temporary wells) on spots comprised on the surface of the solid carrier.
Figure 4: An example of evaluated data resulting from the application of fluorescence microscopy and a cell counting method of a cell distribution on a surface of a solid carrier of a cell array, wherein the cells have been provided without the use of a mesh on the left (standard workflow) and of a cell array according to the present invention on the right (improved workflow).

### List of Reference Numerals

- 1: cell array
- 2: solid carrier
- 3: first mesh
- 4: second mesh

## Claims

1. A cell array comprising a solid carrier comprising on a surface a plurality of spots having a spot size X, and cells on the surface of the solid carrier, **characterized in that** the solid carrier comprises 9 to 400 spots per cm².

2. The cell array according to claim 1, wherein the cells on the surface of the solid carrier have a substantially uniform distribution.

3. The cell array according to claim 1 or 2, wherein the cells are provided onto the surface of the solid carrier by the following steps in the order:
(a) providing a first mesh on the surface of the solid carrier comprising the plurality of spots having the spot size X, wherein the first mesh has a mesh size M1 of n·0.8X ≤ M1 ≤ n·1.2X, wherein n is a natural number of at least 1;
(b) providing a second mesh on the first mesh, wherein the second mesh has a mesh size M2 which is smaller than the mesh size M1 of the first mesh;
(c) providing cells onto the second mesh and thereby bringing the cells into contact with the surface of the solid carrier; and
(d) removing the first mesh and the second mesh.

4. The cell array according to any one of claims 1 to 3, wherein the spot size X is from 5 to 750 µm in diameter.

5. The cell array according to claim 3 or 4, wherein M2 is ≤ 0.5M1.

6. The cell array according to any one of claims 3 to 5, wherein the surface of the first mesh and the surface of the second mesh comprises at least one cell-repelling material.

7. A manufacturing method of a cell array, wherein the method comprises the following steps in the order:
(a) providing a first mesh on a surface of a solid carrier containing a plurality of spots having a spot size X, wherein the first mesh has a mesh size M1 of n·0.8X ≤ M1 ≤ n·1.2X, wherein n is a natural number of at least 1;
(b) providing a second mesh on the first mesh, wherein the second mesh has a mesh size M2 which is smaller than the mesh size M1 of the first mesh;
(c) providing cells onto the second mesh and thereby bringing the cells into contact with the surface of the solid carrier; and
(d) removing the first mesh and the second mesh.

8. The manufacturing method according to claim 7, wherein step (d) is carried out 3 to 5 hours after step (c).

9. Use of the cell array according to any one of claims 1 to 6 for solid phase transfection, wherein each of the plurality of spots comprises a transfection mixture comprising a transfection agent, at least one protein and/or at least one nucleic acid molecule, and the spots are brought into contact with the cells on the surface of the solid carrier.
